(19) Europäisches Patentamt
European Patent Office
Office européen des brevets

(11) Publication number: 0 038 619
B1

(12) EUROPEAN PATENT SPECIFICATION

(45) Date of publication of patent specification: 05.12.84

(21) Application number: 81300977.6

(22) Date of filing: 09.03.81

(51) Int. Cl.$^3$: C 07 D 461/00, C 07 D 471/20 // (C07D471/20, 221/00, 209/00, 209/00)

# (54) Process for preparing vincamine and related alkaloids.

(30) Priority: 17.04.80 IT 2145980

(43) Date of publication of application: 28.10.81 Bulletin 81/43

(45) Publication of the grant of the patent: 05.12.84 Bulletin 84/49

(84) Designated Contracting States: BE DE FR GB

(56) References cited:
BE-A- 761 628
BE-A- 870 978
FR-A-2 358 412

The file contains technical information submitted after the application was filed and not included in this specification

(73) Proprietor: INVERNI DELLA BEFFA S P A
Via Ripamonti, 99
Milan (IT)

(72) Inventor: Bombardelli, Ezio
99 Via Ripamonti
Milan (IT)
Inventor: Gabetta, Bruno
99 Via Ripamonti
Milan (IT)

(74) Representative: Ritter, Stephen David et al
Mathys & Squire 10 Fleet Street
London EC4Y 1AY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 038 619 B1

**Description**

The invention relates to the conversion, with high yields and by a simple route, of (—)vincadifformine and related compounds into (+)vincamine, 16-epivincamine, apovincamine and alkaloids of related structure.

Processes for the conversion of (—)vincadifformine (a product obtainable by reducing tabersonine) into vincamine and correlated alkaloids are already known.

For example, Belgian Patent Specification No. 761.628 of Omnium Chimique describes the conversion of tabersonine into vincamine by treating the reduction product of tabersonine, i.e. (—)vincadifformine, with per-acids as oxidizing agents, with subsequent treatment with reducing agents and acids. A subsequent patent achieves the same conversion using oxygen in the presence of chromium, cobalt and copper salts. Other processes (Parcor, French Patent Specification No. 2358412, Buskine, Netherlands Patent Application No. 7509118) employ oxygen as oxidizing agent acting on the anion of the vincadifformine. A further process (Boehringer, Belgian Patent No. 870978) carries out the same conversion by utilizing hydrogen peroxide in the presence of soluble salts of the hydrated oxides of vanadium, chromium, molybdenum and tungsten.

The results which can be achieved with all these processes are, in one way or another, unsatisfactory. In some cases, the yields obtained in practice are distinctly lower than those reported in the Examples of the Patent Specifications, or the products are difficult to purify. Also, some processes involve environmental disadvantages due to the presence of metallic salts (copper, chromium, etc.) in the effluents; and finally, reagents of very high cost sometimes need to be employed.

The present invention provides a very simple and economic process which enables vincamine (and alkaloids of related structure) to be obtained in high yields and in a state of remarkable purity.

According to the present invention, there is provided a process for converting (—)vincadifformine or a compound of related structure to (+)vincamine, 16-epivincamine, apovincamine or an alkalid of related structure characterised in that a solution of (—)vincadifformine or of a compound of related structure in an acid reaction medium is contacted with ozone, the resulting product is treated with base and the alkaloid(s) so-obtained are isolated.

The term "products of the related structure to (—)-vincadifformine" as used herein refers to compounds containing the same nucleus as (—)vincadifformine, i.e. a nucleus of the structure:—

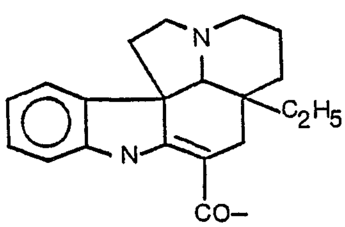

The nucleus may be substituted by one or more groups which do not interfere with the ozonization reaction e.g. $CH_3O$ groups; and the —CO— group may form part of an ester group other than a methyl ester, or a free —COOH group. Similarly, various isomeric forms may be used, e.g. (+)vincadifformine and also salts of the free alkaloid.

Thus, for example, the starting material may have the formula

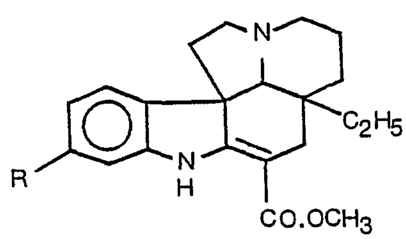

(I)

in which R represents hydrogen or methoxy, whereupon ozonization of the reaction mixture, and the subsequent alkalization, leads to products of the following structural formulae:

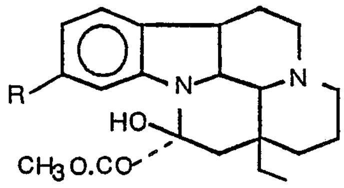

(II)

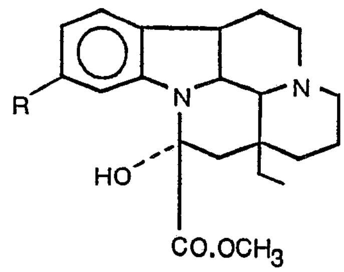

(III)

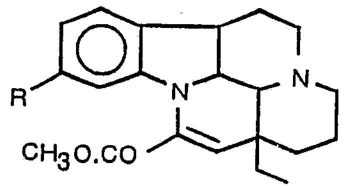

(IV)

in which R has the above-indicated significance. The compounds obtained can be separated from one another by means of chromatography or by equivalent methods.

If the process is performed starting from (—)-vincadifformine (I, with R = H), there will be obtained in practice a mixture of vincamine, 16-epivincamine and apovincamine (II, III and IV, respectively, with R = H), with a distinct preponderance of vincamine. Where R in the starting material is $CH_3O$ as in 11-methoxy-(—)-vincadifformine (V)

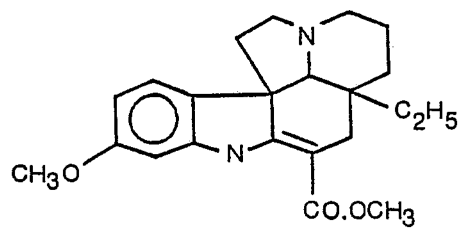

(V)

the major product of the process of the invention will be 11-methoxy -(+)-vincamine (II, with R = $CH_3O$—) otherwise known as vincine.

The process of the invention may be carried out with or without isolation of intermediate products formed as a result of the ozonization. Thus for example 16-hydroxyvincadifformine (or products of related structure) may be isolated as an intermediate product, particularly where the ozonization is carried out at relatively low temperatures, e.g. around 0°C. This intermediate product may then be converted to the desired final product by treatment with an acid, particularly by heating in the presence of an acid.

In general, the contacting with ozone can be carried out by dissolving the (—)vincadifformine (or related compounds) in dilute mineral acids, or in carboxylic acids compatible with the ozonization process, or in alcohols such as methanol, and causing the mixture to absorb the amount of ozone necessary for completion of the conversion. The ozonization may take place at temperatures preferably between 0°C and +70°C when the operation is performed in aqueous medium, or between —70 and +70°C when the operation is performed in organic medium. The reaction times depend on the temperature and on the rate of introduction and of absorption of $O_3$.

After complete conversion of the (—)-vincadifformine (or related compounds), the reaction mixture is brought to, or maintained at, a temperature between 30°C and 70°C for, for example, between 10 minutes and 1 hour. Following this, treatment of the resulting solution with a base and subsequent extraction with solvents immiscible with water, such as for example, ethers, alcohols or chlorinated hydrocarbons, a product mixture is obtained, from which the individual components may be obtained in a pure state by, for example, chromatographic methods or fractional crystallization.

By using the process of the invention and operating under appropriate conditions, for example in a methanol solution and at a temperature between —30 and 10°C, direct crystallization of pure (+)-vincamine, in very high yields, is obtained, after alkalization and concentration, by simple cooling. Compared to the prior art, this constitutes a significant technical advance. A further advantage of the process of the invention is that it enables, in practice, the solutions of (—)-vincadifformine which are obtained by hydrogenation of tabersonine to be subjected directly to the treatment with ozone without isolation of intermediates.

The following Examples serve to illustrate the invention.

Example 1

2 grams (5.91 mmoles) of (—)-vincadifformine are dissolved in 143ml of 0.65N solution of sulphuric acid and treated with 6.5 mmoles of ozone, at 65—70°C, for a period of 20 minutes. The red solution is then maintained at this temperature for another 20 minutes.

The reaction mixture is cooled to room temperature and made alkaline with ammonia to pH 8.5. The partially precipitated alkaloids are extracted three times with 50 ml of methylene chloride. The combined organic extracts are concentrated to dryness and the residue is taken up in 35 ml of methanol. The solution is left over night in a refrigerator and 885 mg of (+)vincamine are obtained (yield (42.1%).

The mother liquors of crystallization are concentrated to dryness and chromatographed on silica gel, the alkaloids being eluted with a mixture of methylene chloride and methanol with a varying gradient of concentrations.

The elution is initiated with pure methylene chloride. The first fraction contains 50 mg of apovincamine (yield 2.5%) and a subsequent fraction contain (+)-vincamine (150 mg) (yield 7.2%). There are also obtained, in a subsequent fraction, 430 mg of 16-epivincamine (yield 20.6%).

The UV, IR, NMR and MS spectra of the products are identical to those of authentic samples and the same is true for the normal physiochemical tests.

Example 2

1.4 grams of (—)-vincadifformine are dissolved in 100 ml of 1N acetic acid and 1N sodium acetate (pH 3.75) and are ozonized at a temperature of 25°C until the starting alkaloid had completely disappeared.

The reaction mixture is heated for 1 hour at 70°C, subsequently treated with ammonia to bring the pH to 8.5 and extracted wtih methylene chloride.

1.4 g of a mixture of vincamine and 16-epivincamine are obtained and by crystallization of the residue, 0.72 g of pure vincamine is obtained. By a chromatographic procedure, 0.02 g of vincamine and 0.3 g of 16-epivincamine are obtained.

Example 3

1.7 grams of (—)-vincadifformine are dissolved in 100 ml of 1N acetic acid and 1N sodium acetate at pH 3.75 and are ozonized at a temperature of 0°C until the starting alkaloid had completely disappeared. At the end of the reaction, the reaction mixture is made alkaline with NaOH to pH 8.5 and extracted three times using 50 ml of methylene chloride each time. On evaporation to dryness under vacuum, the organic phase yields an amorphous product which, after chromatography on silica gel and elution with benzene and triethylamine in a ratio of 85:5, yields 1.07 g of 16-hydroxyvincadifformine. The structure of the compound is confirmed by the IR, NMR, MS and UV data.

Example 4

1.05 grams of 16-hydroxyvincadifformine are dissolved in 50 ml of methanol and 1.5 ml of 96% sulphuric acid are added; the solution is heated for 20 minutes with weak reflux. On disappearance of the 16-hydroxyvincadifformine and after neutralization of the solution with ammonia, the reaction mixture is diluted with 50 ml of $H_2O$ and extracted three times with 50 ml of methylene chloride at a time. After evaporation of the solvent to dryness, the residue is crystallized from methanol and after crystallization and chromatography of the mother liquors, 0.720 g of vincamine and 0.3 g of 16-epivincamine are obtained.

Example 5

20 grams (59.1 mmoles) of (—)-vincadifformine are dissolved in 100 ml of methanol to which 7.092 g (118.2 moles) of glacial acetic acid are added. At a temperature maintained at —20°C, the solution is caused to absorb an equimolar amount of $O_3$ in the course of about 3 hours. The solution is then brought to room temperature and after about half an hour it is brought to pH 7 with concentrated $NH_4OH$.

40 ml of methanol are eliminated by distillation and then the residue is cooled to 0°C. Following filtration using a pump, 15.17 g (71%) of pure (+)vincamine are directly obtained. Smaller amounts of (+)-vincamine and, moreover, apovincamine and 16-epivincamine are obtained by treatment of the mother liquors in the manner set forth in the preceding Examples.

Similar results as regards yield and purity of the product are obtained by replacing the acetic acid by a corresponding amount of sulphuric acid, or by operating directly on methanol solutions of (—)-vincadifformine obtained by hydrogenation of tabersonine.

It will be appreciated that in the above Examples compounds of related structure may be used instead of (—)-vincadifformine as starting material to produce corresponding alkaloids of related structure to (+)-vincamine, 16-epivincamine or apovincamine. Such alkaloids will in general contain a nucleus of the structure

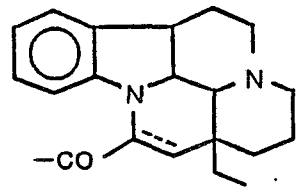

**Claims**

1. A process for converting (—)-vincadifformine or a compound of related structure to (+)-vincamine, 16-epivincamine, apovincamine or an alkaloid of related structure characterised in that a solution of a compound containing a nucleus of the formula

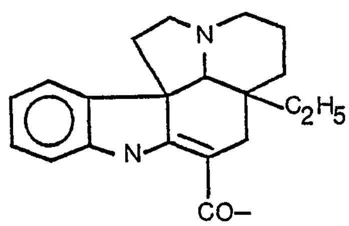

in an acid reaction medium is contacted with ozone, the resulting product is treated with base and the alkaloid(s) so-obtained are isolated.

2. A process according to Claim 1 wherein the starting material has the formula

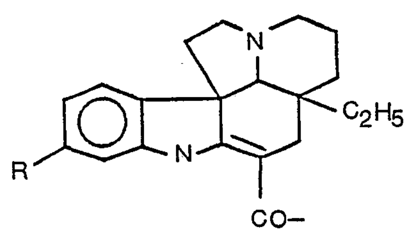

wherein R is hydroxy or methoxy and the —CO— group forms part of an ester group or a —COOH group.

3. A process according to Claim 1 wherein the starting material is (—)-vincadifformine and the products include one or more of (+)-vincamine, 16-epivincamine and apovincamine.

4. A process according to Claim 1 wherein the starting material is 15-methoxy-(—)-vincadifformine and the products include 11-methoxy-(+)-vincamine (vincine).

5. A process according to any preceding claim wherein the ozonization is effected in an organic solvent at between about —70 and +70°C.

6. A process according to Claim 5 in which the organic solvent comprises one or more carboxylic acids compatible with ozone.

7. A process according to Claim 5 in which the organic solvent comprises methanol.

8. A process according to any one of Claims 1 to 4 in which the ozonization is effected in an aqueous medium at between about 0 and +70°C.

9. A process according to any preceding claim in which 16-hydroxyvincadifformine or a compound of related structure is isolated as an intermediate product and subsequently converted to the desired final product by heating in the presence of an acid.

10. A process according to any preceding claim wherein the starting material comprises an acidified solution of (—)-vincadifformine obtained by hydrogenation of tabersonine and said solution is subjected directly to ozonization.

**Patentansprüche**

1. Verfahren zur Überführung von (—)-Vincadifformin oder einer Verbindung verwandter Struktur in (+)-Vincamin, 16-Epivincamin, Apovincamin oder ein Alkaloid verwandter Struktur, dadurch gekennzeichnet, daß eine Lösung einer Verbindung, die einen Kern der Formel

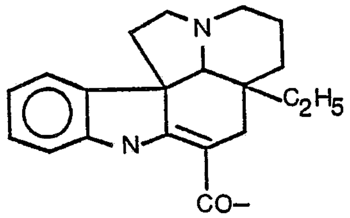

enthält, in einem sauren Reaktionsmedium mit Ozon in Behrührung gebracht wird, das anfallende Produkt mit einer Base behandelt wird und das (die) so erhaltene(n) Alkaloid(e) isoliert wird (werden).

2. Verfahren nach Anspruch 1, worin das Ausgangsmaterial die Formel

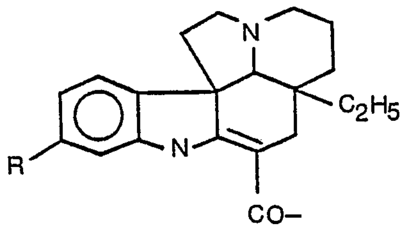

besitzt, worin R Hydroxy oder Methoxy ist und die —CO—Gruppe einen Teil einer Estergruppe oder einer —COOH—Gruppe bildet.

3. Verfahren nach Anspruch 1, worin das Ausgangsmaterial (—)Vincadifformin ist und die Produkte eine oder mehrere der Verbindungen (+)-Vincamin, 16-Epivincamin und Apovincamin umfassen.

4. Verfahren nach Anspruch 1, worin das Ausgangsmaterial 15-Methoxy-(—)-vincadifformin ist und die Produkte 11-Methoxy-(+)-vincamin (Vincin) umfassen.

5. Verfahren nach einem der voranstehenden Ansprüche, worin die Ozonisierung in einem organischen Lösungsmittel zwischen etwa —70 und +70°C durchgeführt wird.

6. Verfahren nach Anspruch 5, worin das organische Lösungsmittel ein oder mehrere mit Ozon verträgliche Carbonsäuren enthält.

7. Verfahren nach Anspruch 5, worin das organische Lösungsmittel Methanol enthält.

8. Verfahren nach einem der Ansprüche 1 bis 4, worin die Ozonisierung in einem wässrigen Medium bei Temperaturen zwischen etwa 0 und 70°C durchgeführt wird.

9. Verfahren nach einem der voranstehenden Ansprüche, worin 16-Hydroxyvincadifformin oder eine Verbindung verwandter Struktur als ein Zwischenprodukt isoliert wird und anschließend durch Erhitzen in Gegenwart einer Säure in das gewünschte Endprodukt überführt wird.

10. Verfahren nach einem der voranstehenden Ansprüche, worin das Ausgangsmaterial eine angesäuerte Lösung von (—)-Vincadifformin enthält, das durch Hydrierung von Tabersonin erhalten wurde, und die Lösung direkt der Ozoniserung unterworfen wird.

**Revendications**

1. Procédé pour convertir la (—)vincadifformine ou un composé de structure apparentée, en (+)vincamine, 16-épivincamine, apovincamine ou un alcaloïde de structure apparentée, caractérisé en ce qu'une solution d'un composé contenant un noyau représenté par la formule ci-après,

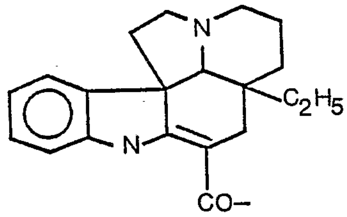

dans un milieu de acide de réaction, est mis en contact avec de l'ozone, que le produit résultant est traité avec une base et que le ou les alcaloïdes ainsi obtenus sont isolés.

2. Procédé suivant la revendication 1, caractérisé en ce que la matière de départ est représentée par la formule ci-après:

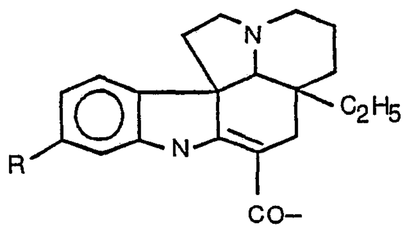

dans laquelle R est un groupe hydroxy ou méthoxy et le groupe —CO— forme une partie d'un groupe ester ou d'un groupe —COOH.

3. Procédé suivant la revendication 1, caractérise en ce que la matière de départ est la (—)vincadifformine et que les produits comprennet un ou plusieurs composés parmi la (+)vincamine, la 16-épivincamine et l'apovincamine.

4. Procédé suivant la revendication 1, caractérisé en ce que la matière de départ est la 11-méthoxy-(-)vincadifformine et que les produits comprennent la 11-méthoxy-(+)-vincamine (vincine).

5. Procédé suivant l'une quelconque des revendications précédentes, caractérisé en ce que l'ozonisation est effecutée dans un solvant organique entre environ —70 et +70°C.

6. Procédé suivant la revendication 5, caractérisé en ce que le solvant organique comprend un ou plusieurs acides carboxyliques compatibles avec l'ozone.

7. Procédé suivant la revendication 5, caractérisé en ce que le solvant organique est le méthanol.

8. Procédé suivant l'une quelconque des revendications 1 à 4, caractérisé en ce que l'ozonisation est effectuée dans un milieu aqueux, entre environ 0° et +70°C.

9. Procédé suivant l'une quelconque des revendications précédentes, caractérisé en ce que la 16-hydroxyvincadifformine ou un composé de structure apparentée, est isolé comme produits intermédiaires et ensuite converti en produit final désiré par chauffage en présence d'un acide.

10. Procédé suivant l'une quelconque des revendications précédentes, caractérisé en ce que la matière de départ comprend une solution acidifiée de (—)vincadifformine obtenue par l'hydrogénation de tabersonine et que cette solution est directement soumise à l'ozonisation.